# FASCICULE DE BREVET EUROPEEN

(11) **EP 3 834 884 B1**
(45) Date de publication et mention de la délivrance du brevet: **31.08.2022**
(21) Numéro de dépôt: 20211917.8
(22) Date de dépôt: 04.12.2020
(51) Int. Cl.: A61N 5/06, A61N 1/05

(54) **DISPOSITIF D'ILLUMINATION IMPLANTABLE DANS UN ÊTRE VIVANT**
IN EIN LEBEWESEN IMPLANTIERBARE BELEUCHTUNGSVORRICHTUNG
ILLUMINATION DEVICE IMPLANTABLE IN A LIVING BEING

(30) Priorité: 12.12.2019 FR 1914298
(43) Date de publication de la demande: 16.06.2021
(73) Titulaire: Commissariat à l'Energie Atomique et aux Energies Alternatives, 75015 Paris (FR)
(72) Inventeur: CHABROL, Claude, 38054 GRENOBLE cedex 09 (FR)
(74) Mandataire: INNOV-GROUP

(56) Documents cités:
- WO-A1-2014/135197
- US-A1- 2015 231 408

## Description

### Domaine technique de l'invention

La présente invention se rapporte à un dispositif d'illumination destiné à être implanté au moins en partie dans un être vivant pour illuminer, de manière localisée, au moins une zone de l'être vivant. L'invention concerne également le module à stimulation optique qui permet de réaliser facilement une sonde d'un tel dispositif d'illumination.

### Etat de la technique

Pour le traitement de certaines pathologies d'un être vivant, il a été imaginé d'aller stimuler de manière optique une zone interne d'un être vivant. Pour cela, il a été proposé des dispositifs qui comportent une source de lumière et qui sont implantés au moins en partie ou totalement dans l'être vivant pour aller illuminer la zone souhaitée.

On s'est notamment aperçus de l'intérêt de tels dispositifs pour illuminer/irradier de manière optique certaines zones du cerveau humain.

Cependant, compte-tenu des risques liés à l'implantation d'un tel dispositif dans le cerveau, on comprend qu'un tel dispositif doive être parfaitement conçu.

La demande de brevet US2017281928A1 et le brevet US10213596B2 décrivent des dispositifs d'illumination implantables comprenant un générateur d'impulsions (IPG pour "Implantable Pulse Generator") qui alimente une source lumineuse et une sonde comportant un guide lumière chargé d'amener un faisceau lumineux vers la zone à traiter.

Ces solutions ne répondent pas aux critères suivants :
- Illumination sur une longueur adaptable de 2 à 50mm ;
- Compatibilité d'une source d'illumination totale avec des générateurs de type IPG disponibles sur le marché (c'est-à-dire sans modification "hardware") ;
- Modularité (possibilité de stimuler de manière électrique et/ou optique, choix de plusieurs longueurs d'ondes d'illumination...) ;

Le document WO 2014/135197 divulgue une sonde implantable comprenant les modules qui contiennent les diodes lumineuses connectées tête-bêche pour éviter les fuites de courant.

Le document US 2015/231408 décrit une sonde à stimulation optique, étant destinée à comporter une chaîne formée de plusieurs de modules, ledit module comportant :
des premiers contacts électriques dits amont, agencés sur son boîtier pour se connecter à un premier module à stimulation optique identique adjacent, situé en amont dans une chaîne de modules, et des deuxièmes contacts électriques dits avals agencés sur son boîtier pour se connecter à un deuxième module à stimulation optique identique adjacent, situé en aval dans ladite chaîne de modules,
- des liaisons électriques agencés entre chaque premier contact électrique amont et chaque deuxième contact électrique aval,
- un premier contact électrique d'alimentation dédié, agencé sur son boîtier sur lequel est connecté son bloc électronique.

La sonde n'est pas implantable.

Le but de l'invention est de proposer un module à stimulation optique pouvant être facilement intégré dans une sonde d'un dispositif d'illumination, ladite sonde étant destinée à être implantée au moins en partie dans un être vivant, notamment pour illuminer une ou plusieurs zones de son cerveau. Le module présente une architecture adaptée permettant à la sonde de remplir les différents critères évoqués ci-dessus. Le module peut notamment être facilement intégré à la sonde et cascadé dans ladite sonde, sans modification de son architecture.

### Exposé de l'invention

Ce but est atteint par un module à stimulation optique à intégrer dans une sonde implantable dans un être vivant pour illuminer de manière localisée une zone dudit être vivant, ladite sonde étant destinée à comporter une chaîne formée de plusieurs de ces modules, caractérisé en ce qu'il comporte :
- un boîtier,
- un bloc électronique hermétique logé dans ledit boîtier et comportant deux diodes lumineuses connectées tête bêche, et
- des premiers contacts électriques dits amont, agencés sur son boîtier pour connecter un premier module à stimulation optique identique adjacent, situé en amont dans une chaîne de modules, et des deuxièmes contacts électriques dits avals agencés sur son boîtier pour connecter un deuxième module à stimulation optique identique adjacent, situé en aval dans ladite chaîne de modules,
- des liaisons électriques continues agencées entre chaque premier contact électrique amont et chaque deuxième contact électrique aval,
- un contact électrique d'alimentation dédié, agencé sur son boîtier sur lequel est connecté son bloc électronique.

Selon une particularité, lesdites liaisons électriques comportent au moins une liaison formant une ligne de retour électrique commune à tous les modules de la sonde, sur laquelle est connecté ledit bloc électronique du module.

Selon une particularité, le bloc électronique comporte au moins un substrat comportant deux faces opposées, lesdites deux diodes lumineuses étant montées sur une seule des deux faces dudit substrat.

Selon une autre particularité, le bloc électronique peut comporter un capot hermétique adapté sur le substrat.

Selon une autre particularité, le bloc électronique peut comporter un dépôt de type ALD venant recouvrir les deux diodes lumineuses.

Selon une autre particularité, le bloc électronique peut comporter deux substrats, sur chacun desquels est montée une diode lumineuse distincte.

Selon une autre particularité, le boîtier du module peut comporter une bague fermée à ses deux extrémités par deux bouchons, lesdits deux bouchons portant des moyens de support du bloc électronique logé dans le boîtier.

Selon une autre particularité, le module peut comporter des électrodes de stimulation sur la surface latérale de sa bague.

Selon une autre particularité, le module peut comporter un matériau d'enrobage injecté dans son boîtier autour du bloc électronique hermétique.

L'invention concerne également une sonde implantable dans un être vivant destinée à être connectée électriquement à une source d'alimentation électrique et présentant une architecture allongée, caractérisée en ce qu'elle comporte plusieurs modules à stimulation optique juxtaposés le long de la sonde et séparés entre eux d'une distance non nulle, ladite sonde comprenant un matériau d'enrobage venant combler l'espace entre deux modules adjacents, chaque module à stimulation optique étant tel que défini dans ci-dessus, ladite sonde comportant plusieurs voies électriques destinées chacune à être raccordé électriquement en point à point à une voie électrique distincte de la source d'alimentation électrique, chaque module à stimulation optique de la sonde étant connectée en série sur une voie électrique distincte de la sonde via son contact électrique d'alimentation dédié.

Selon une particularité, la sonde comporte une armature formée d'un câble sur lequel sont enfilées lesdits modules.

Selon une autre particularité, entre deux modules adjacents, la sonde comporte une bague de séparation mécanique, enfilée sur ladite armature.

Selon une autre particularité, la sonde comporte un module à illumination axiale situé à proximité de l'extrémité distale de la sonde.

L'invention concerne également un dispositif d'illumination implantable, destiné à être implanté dans un être vivant pour illuminer de manière localisée une zone dudit être vivant, ledit dispositif comportant une source d'alimentation électrique comportant plusieurs voies d'alimentation électrique en parallèle et une sonde connectée électriquement à la source d'alimentation électrique et présentant une architecture allongée entre une extrémité proximale et une extrémité distale, ladite sonde étant telle que définie ci-dessus.

Selon une particularité, la source d'alimentation électrique est un générateur d'impulsions implantable.

### Brève description des figures

D'autres caractéristiques et avantages vont apparaître dans la description détaillée qui suit faite en regard des figures annexées et listées ci-dessous :
- La figure 1 représente un exemple de réalisation du dispositif conforme à l'invention ;
- Les figures 2A et 2B représentent l'architecture électrique du dispositif de l'invention, selon deux exemples de réalisation ;
- Les figures 3A et 3B illustrent différents types de signaux de tension pouvant être émis par le générateur employé dans le dispositif de l'invention ;
- Les figures 4A et 4B représentent un premier exemple de réalisation de la sonde conforme à l'invention ;
- Les figures 5A et 5B représentent, respectivement en vue de côté et en vue axiale, un module employé dans le dispositif de l'invention, selon le premier exemple de réalisation des figures 4A et 4B ;
- Les figures 6A et 6B représentent un deuxième exemple de réalisation de la sonde conforme à l'invention, respectivement suivant une vue schématique de côté et suivant une vue en coupe transversale selon A-A ;
- Les figures 7A et 7B représentent, respectivement en vue de côté et selon la coupe B-B, le module employé dans le dispositif de l'invention, selon le deuxième exemple de réalisation des figures 6A et 6B ;
- La figures 8A représente un mode de réalisation préféré de la sonde du dispositif de l'invention ;
- La figure 8B illustre une variante de réalisation de l'architecture de la figure 8A ;
- Les figures 9A à 9D représentent quatre modes de réalisation du support des diodes lumineuses qui est employé dans le dispositif de l'invention ;
- Les figures 10A à 10D illustrent différentes configurations d'illumination permises par le dispositif de l'invention ;
- Les figures 11A à 11E représentent des exemples de réalisation des électrodes présentes sur la surface du module ; La figure 11A est une vue de côté en coupe longitudinale ; Les figures 11B, 11C et 11D, sont des vues en coupe transversale et la figure 11E une vue de dessus ;
- La figure 12 illustre de manière schématique les différents types de modules pouvant être employé dans la sonde de l'invention ;
- Les figures 13A à 13C représentent trois exemples de réalisation d'une sonde hybride pouvant être employée dans le dispositif de l'invention ;
- La figure 14 représente un autre mode de réalisation de la sonde de l'invention ;

### Description détaillée d'au moins un mode de réalisation

L'invention concerne un dispositif 1 implantable d'illumination cérébrale profonde (DBS - "Deep Brain Stimulation"). Ce dispositif permet notamment de réaliser une illumination localisée (par exemple dans le proche infra-rouge ou avec toute autre longueur d'onde selon le traitement envisagé - traitement type neuroprotection, optogénétique) des tissus cibles (par exemple SNc, hippocampe, striatum...) tout en minimisant les risques médicaux lors de l'implantation. Ce dispositif peut servir en particulier dans le traitement de maladies neurodégénératives telles que la maladie de Parkinson, Alzheimer, Huntington...

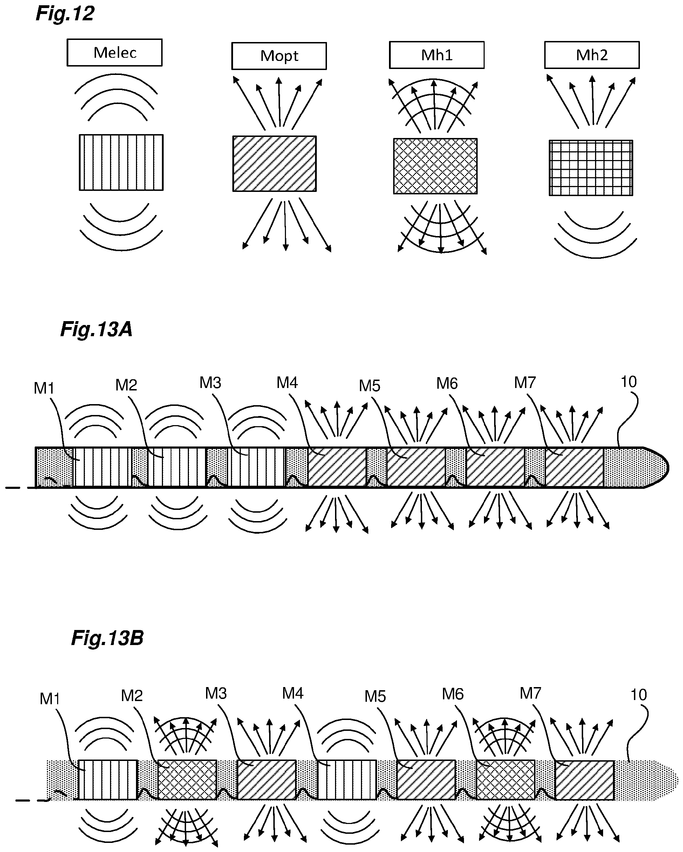

On verra que le dispositif 1 peut éventuellement intégrer des solutions proposant d'autres modes de stimulations (électrique, injection...). Les figures 13A à 13C illustrent le principe de réalisations hybrides.

L'illumination des tissus peut avoir différents objectifs selon l'application : neuroprotection, optogénétique, stimulation... Plusieurs cibles sont concernées, par exemple : la substance noire compacta (SNc) qui dégénère dans la maladie de Parkinson, l'hippocampe, principal noyau mis en cause dans la maladie d'Alzheimer, le striatum pour la maladie d'Huntington. L'illumination peut être apportée directement dans les tissus (avec risque de lésions supplémentaires) ou en choisissant des trajectoires passant par les ventricules (cavités permettant la circulation du liquide céphalorachidien LCR) et en contact avec les structures à traiter (illuminateur directifs).

Le dispositif 1 de l'invention comporte une source d'alimentation électrique. Cette source d'alimentation est avantageusement composée d'un générateur d'impulsions implantable (appelé couramment IPG pour "Implantable Pulse Generator"), référencé IPG sur les dessins.

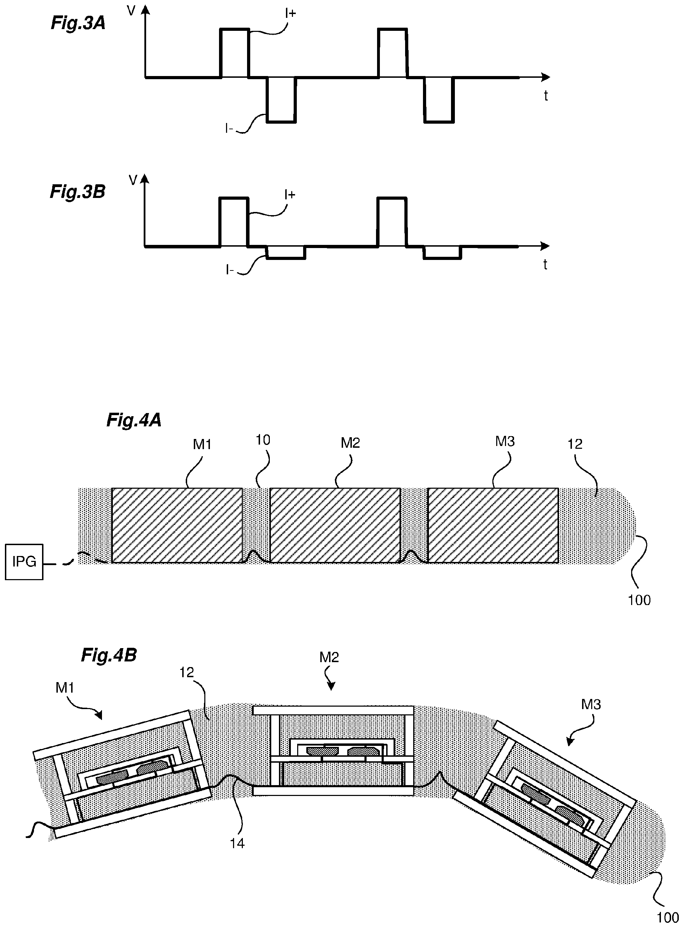

De manière connue, un générateur d'impulsions implantable IPG comporte principalement une carte électronique et une batterie, rechargeable ou non. La carte électronique comporte un microcontrôleur chargé de gérer le fonctionnement du générateur. Le générateur IPG peut notamment être programmé pour fournir des impulsions dites bipolaires, telles que celles représentées sur les figures 3A et 3B. Sur les figures 3A et 3B, le motif comporte ainsi une impulsion carrée positive I₊ et une impulsion carrée négative I-, séparées entre elles d'un temps mort. Pour équilibrer les charges électriques injectées dans les tissus, les deux impulsions peuvent être symétriques, comme illustré sur la figure 3A, ou modulées en amplitude et en durée, comme illustré sur la figure 3B, avec la même quantité de charge injectée pour les deux impulsions (t x Courant x Tension avec t durée de l'impulsion). Le générateur comporte n voies, avec n supérieur ou égal à 2. Chaque voie est référencée Vi avec i allant de 1 à n.

Le dispositif 1 comporte ensuite une sonde 10 implantable connectée au générateur.

Cette sonde 10 implantable se présente sous la forme d'une tige allongée souple. La sonde 10 présente avantageusement une section transversale circulaire. A titre d'exemple, le diamètre de la section de la sonde peut aller de 1 à 3mm, préférentiellement 1.3mm pour être compatible avec les outils standards utilisés en DBS.

Le dispositif 1 comporte des moyens de connexion électrique permettant de connecter la sonde 10 au générateur IPG par son extrémité proximale, par l'intermédiaire d'un connecteur 15 et d'une extension 16. À l'extrémité distale, la sonde 10 présente avantageusement une forme atraumatique 100 (par exemple avec une forme oblongue ou sphérique).

Selon l'invention, sur au moins une partie de sa longueur, la sonde 10 intègre plusieurs modules de stimulation optique juxtaposées formant une barrette. Cette barrette est avantageusement située à proximité de l'extrémité distale de la sonde 10.

Le nombre de modules de la sonde peut être adapté selon la pathologie à traiter et selon la taille du tissu traité (pouvant aller par exemple de 5 à 50mm de long).

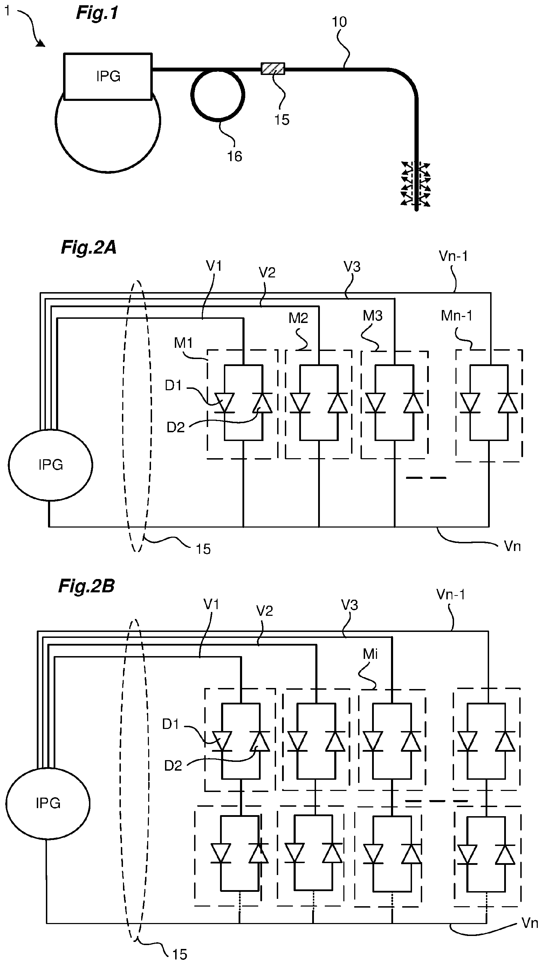

Sur les figures 2A et 2B, les modules à stimulation optique sont référencés Mi, avec i allant de 1 à n-1 avec n supérieur ou égal à 3 (n correspondant au nombre de voies du générateur IPG employé). La sonde comporte ainsi au moins deux modules à stimulation optique connectés en parallèle au générateur IPG. A titre d'exemple, le générateur peut comporter 8 ou 12 voies.

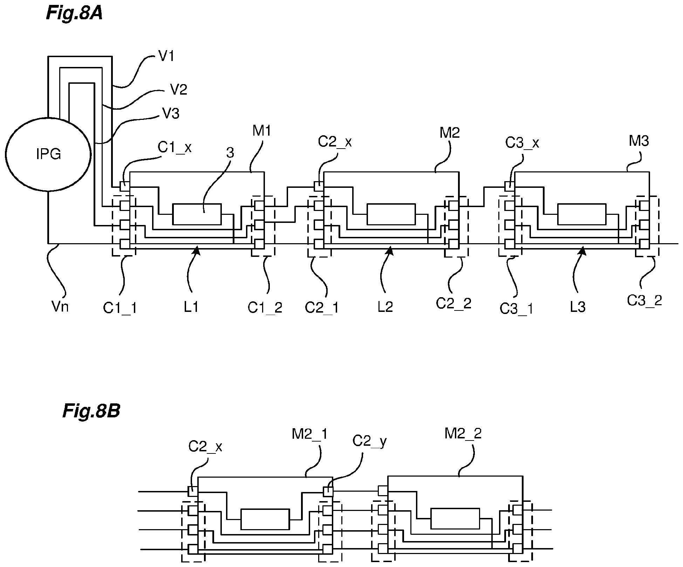

Dans le dispositif, les modules sont connectés en point à point au générateur IPG et peuvent ainsi être adressées individuellement par celui-ci. Chaque module ou série de modules (au moins deux modules câblés en série sur chaque voie comme sur la figure 2B) est ainsi connectée sur une voie Vi distincte du générateur. Une unique liaison de retour permet de reboucler l'ensemble des modules sur une voie du générateur IPG. Un principe de connexion de chaque module dans la chaîne sera explicité ci-après en liaison avec la figure 8A.

En référence aux figures 4A et 4B, dans un premier exemple de réalisation, les modules Mi sont connectés entre eux de manière à former une guirlande et maintenus entre eux par un matériau d'enrobage 12 de type silicone, polyuréthane ou époxy, le matériau pouvant être choisi notamment en fonction de la rigidité visée pour l'application. Chaque module Mi est relié au générateur IPG par une liaison point à point. Un premier conducteur électrique 14 gainé et isolé permet de transporter l'ensemble des voies du générateur IPG vers les modules. Un deuxième conducteur électrique également gainé et isolé peut former la liaison électrique de retour vers le générateur IPG. Toute autre solution de câblage électrique peut être employée.

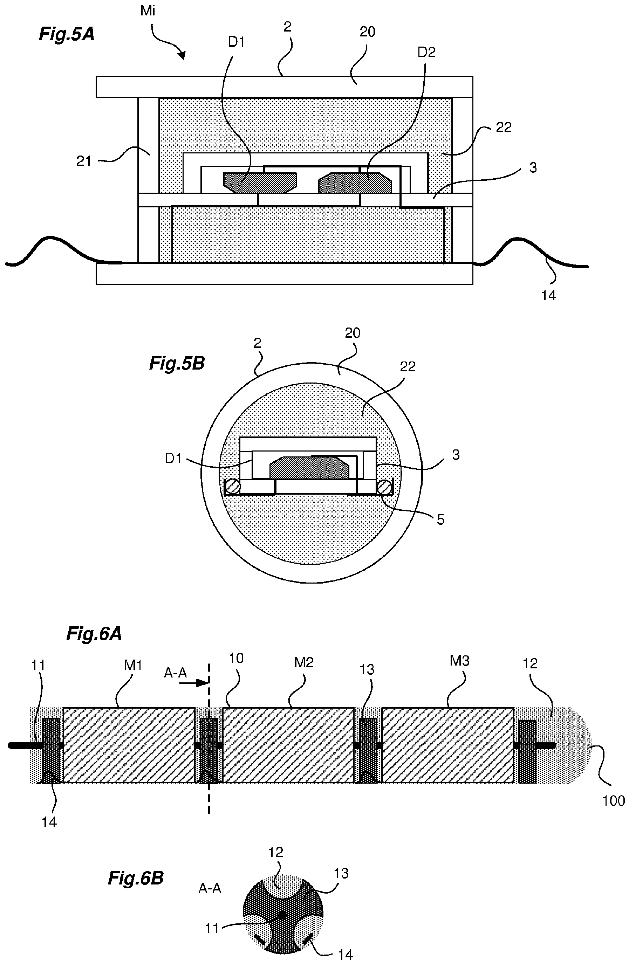

En référence aux figures 6A et 6B, dans un autre exemple de réalisation, la sonde 10 peut comporter une armature 11, par exemple formée d'une âme centrale (rigide ou souple selon le besoin) telle qu'un câble métallique solidaire des modules, l'enrobage 12 de type silicone, polyuréthane ou époxy venant combler l'espace entre modules. L'enrobage en polyuréthane permettra notamment de préformer la sonde à la demande. Le câble formant l'armature 11 permet de limiter l'élongation de la sonde 10 lors de la manipulation du dispositif, notamment lors de son insertion chirurgicale et des différentes étapes de nettoyage lors de la production. Les modules Mi sont séparés entre eux le long de l'armature 11 d'une longueur non nulle, par du matériau d'enrobage 12, de manière à maintenir des zones de flexion et à permettre à la sonde 10 de conserver un certain niveau de flexibilité. Le câble 11 peut par ailleurs, servir de "plan de masse" pour le retour du courant de l'ensemble des pistes d'alimentation.

Les modules sont enfilés sur l'armature 11, placée dans l'axe des modules Mi. Une bague 13 de séparation enfilée sur l'armature 11 peut être positionnée entre deux modules Mi adjacents, pour limiter la flexion appliquée à la sonde dans une plage déterminée, en vue notamment de protéger la connexion électrique et de faciliter la mise en place chirurgicale de l'implant. Comme représenté sur la figure 6B, chaque bague de séparation 13 peut avoir une forme en étoile à plusieurs branches, ce qui permet au matériau d'enrobage de s'écouler entre deux modules pour faire la liaison et de laisser un espace pour le passage des conducteurs 14 d'un module à l'autre.

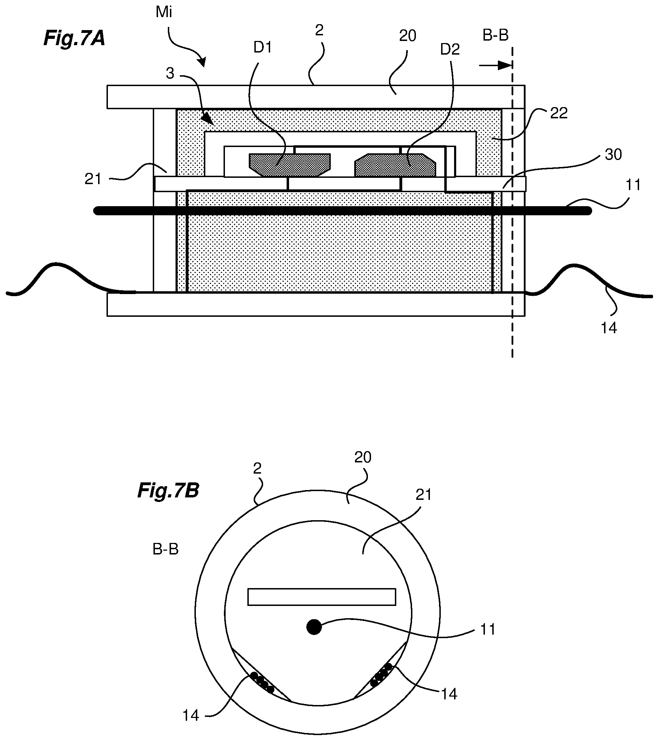

En référence aux figures 5A, 5B, 7A et 7B, chaque module Mi de stimulation optique peut comporter un boîtier 2. Le boîtier 2 peut comporter une bague 20 réalisée dans un matériau transparent et biocompatible tel que le saphir ou la silice et fermée à ses deux extrémités par des bouchons 21, de manière à former un cylindre creux. Le cylindre peut présenter un diamètre externe de 1300µm.

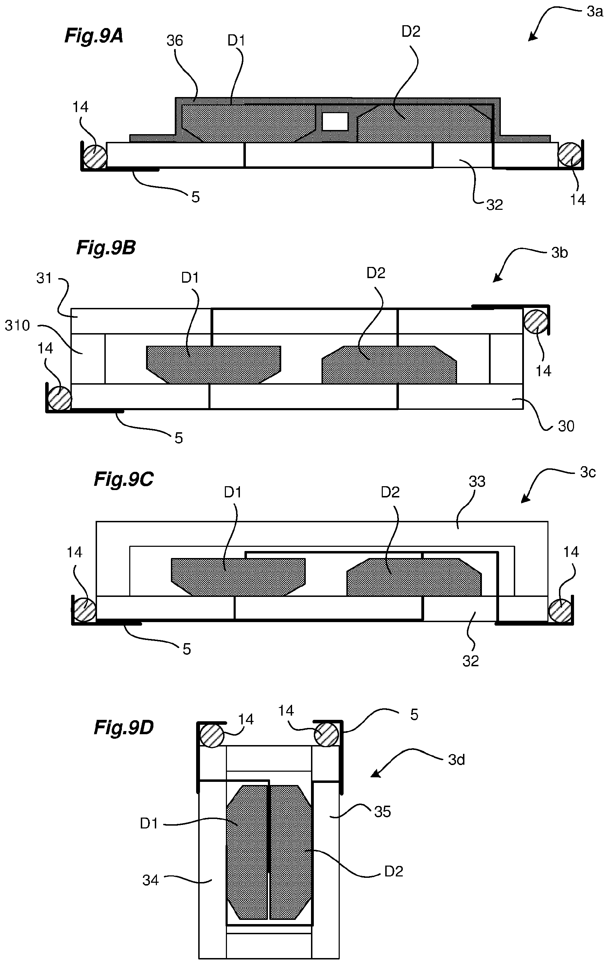

Le module Mi comporte un bloc électronique 3 hermétique logé dans son boîtier. Les deux bouchons 21 servent au bon positionnement du bloc électronique et facilitent le remplissage de la cavité par un matériau d'enrobage 22 transparent ou diffusant selon l'objectif d'uniformité recherché (type silicone, polyuréthane ou époxy). Une colle époxy pourra être sélectionnée avec un indice optique limitant les pertes par réflexion, et pourra également être chargée afin d'améliorer le transfert thermique, l'étanchéité et répondre aux contraintes de dilatation pendant la production (en particulier pour les puces passivées par dépôt ALD - voir ci-après). Comme représenté sur les figures 7A et 7B, les deux bouchons 21 peuvent être percés en leur centre pour laisser passer l'armature 11 de la sonde 10. Dans la sonde, chaque module Mi est ainsi indépendant de chaque autre module et présente sa propre architecture électronique, c'est-à-dire son bloc électronique (voir ci-après en liaison avec les figures 9A à 9D) et ses contacts électriques de connexion, et sa propre architecture mécanique composée de la bague et des bouchons. Il faut noter que l'herméticité du bloc électronique est obtenue au niveau de chaque module, rendant le module facilement intégrable dans une sonde lors de sa fabrication.

Chaque module Mi comporte une source lumineuse logée dans son bloc électronique. La source lumineuse peut comporter au moins deux diodes lumineuses réalisées selon des technologies connues (type LED, OLED, µLED, VCSEL, LASER...). Les diodes peuvent fonctionner à une tension inférieure à quelques volts (2V par exemple pour les LED), avec des courants pouvant aller de 2 à 25mA. Le générateur IPG peut pour sa part fournir une tension de 15V et des courants allant de de 25 à 50mA, ce qui permet par exemple l'alimentation de plusieurs composants en série (exemple jusqu'à sept diodes de 2V). Dans le cas de diodes de type VCSEL (pour "Vertical-Cavity Surface-Emitting Laser") ou LASER, les impulsions fournies par le générateur peuvent être adaptées en durée et en amplitude. L'aire générée par les deux impulsions devra être choisie identique, c'est-à-dire avec la même quantité de charge injectée pour les deux impulsions.

Tous les modules Mi de la sonde sont identiques, notamment au niveau de leurs moyens de connexion électrique. Pour se raccorder chacun à une voie Vi distincte du générateur IPG et sur la voie de retour Vn du générateur IPG, tout en assurant une continuité électrique le long de la chaîne de modules présents dans la sonde, chaque module Mi comporte divers moyens de connexion électrique.

En liaison avec la figure 8A, chaque module Mi (M1, M2, M3 sur la figure 8A) comporte ainsi des premiers contacts électriques Ci_1 dits amont, agencés sur son boîtier 2 pour connecter le module Mi-1, situé en amont dans la chaîne de modules, et des deuxièmes contacts électriques Ci_2 dits avals, agencés sur son boîtier 2 pour connecter le module Mi+1, situé en aval dans ladite chaîne de modules. Des liaisons électriques Li continues (non interrompues) sont agencées pour relier chaque premier contact électrique amont à chaque deuxième contact électrique aval et ainsi assurer une continuité électrique d'un module à l'autre.

Par ailleurs, les moyens de connexion électrique du module Mi comportent un contact électrique d'alimentation Ci_x dédié, agencé sur son boîtier, connecté d'une part en point à point à une seule voie Vi du générateur IPG et d'autre part au bloc électronique 3 du module Mi pour l'alimenter. Parmi lesdites liaisons électriques précitées, l'une d'elles forme la voie de retour Vn sur laquelle vient se connecter le bloc électronique 3 du module Mi. Il faut noter que le nombres de liaisons Li du module détermine le nombre de modules (ou groupes de plusieurs modules en série) que la chaîne est capable d'intégrer. Ce nombre de modules sera au maximum égal au nombre de voies disponibles sur le générateur IPG. Si le générateur IPG comporte 12 voies, chaque module comporte un contact électrique dédié à l'alimentation de son bloc électronique 3 et 11 liaisons électriques Li. Après le premier module M1 de la chaîne, chaque module vient connecter son contact électrique dédié Ci_x sur une liaison distincte disponible en point à point.

Le module M1 de rang 1 dans la chaîne est situé à l'extrémité proximale de la sonde et connecté du côté amont à toutes les voies du générateur IPG.

Des contacts électriques spécifiques peuvent être prévus sur chaque module si le module comporte également des moyens de stimulation électrique.

Dans le cas où deux modules sont connectés en série sur une même voie (comme sur la figure 2B), le premier module de la série peut disposer d'une architecture adaptée. La figure 8B illustre cette réalisation particulière, pour deux modules M2_1, M2_2 connectés en série sur une même voie du générateur. Le premier module M2_1 de la série comporte un contact électrique aval dédié, référencé C2_y, son bloc électronique 3 étant alors connecté directement entre son contact électrique amont C2_x et son contact électrique aval C2_y. Le deuxième module M2_2 de la série est identique au module Mi décrit ci-dessus en liaison avec la figure 8A. Les contacts électriques amonts et avals, ainsi que les liaisons électriques décrites ci-dessus, en liaison avec la figure 8A sont reproduits de manière identique dans les deux modules M2_1 et M2_2 connectés en série.

De manière avantageuse, la source lumineuse de chaque module Mi est composée d'au moins deux diodes lumineuses D1, D2 connectées tête bêche. L'une des deux diodes est ainsi active lors de l'impulsion de tension positive I₊ fournie par le générateur IPG et l'autre est active lors de l'impulsion de tension négative I- fournie par le générateur. Ce montage permet ainsi d'utiliser toute la puissance disponible, tout en protégeant les diodes lumineuses de la tension en inverse.

Dans chaque module Mi, les deux diodes lumineuses D1, D2 peuvent être identiques et émettre à une même longueur d'onde. Mais il est aussi possible de prévoir d'utiliser deux diodes qui émettent à deux longueurs d'onde distinctes, par exemple à 670nm pour l'une et 810nm pour l'autre. Si on souhaite illuminer à partir de deux longueurs d'ondes, il sera utile d'employer des diodes lumineuses de type VCSEL ou de type LASER à la place des diodes lumineuses de type LED, afin d'adapter la puissance de manière indépendante entre les deux longueurs d'ondes, en jouant sur la largeur/amplitude des impulsions positives et négatives fournies par le générateur IPG.

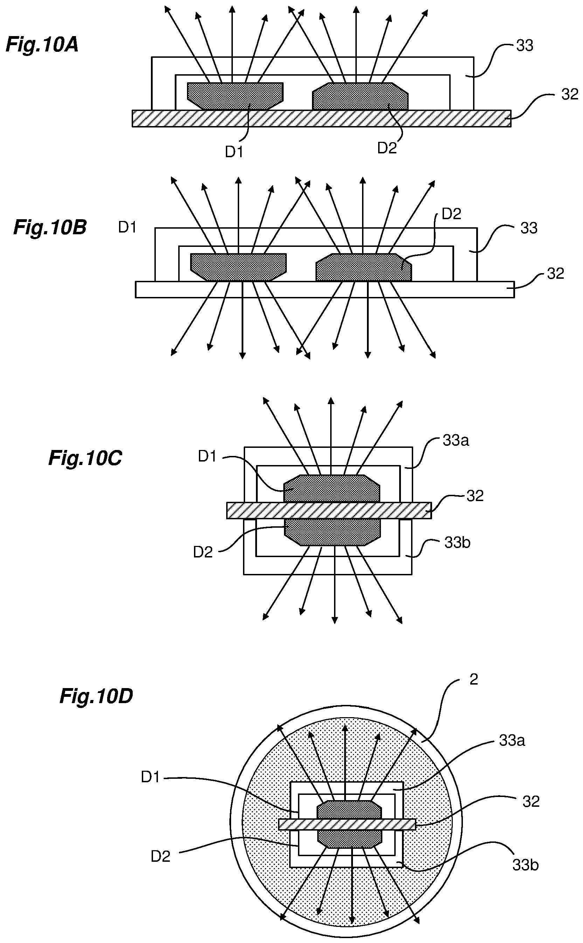

L'illumination est réalisée selon plusieurs directions transversales (notamment radiales pour une sonde à section circulaire) par rapport à l'axe de la sonde. Selon les modules employés, on verra qu'il est possible d'illuminer selon différentes directions, sur toute la périphérie de la sonde ou sur une plage angulaire déterminée plus étroite (voir figures 10A à 10D). Il sera notamment possible d'employer des modules à illumination bidirectionnelle (par exemple 2x110°) ou des modules à illumination plus directive (par exemple 1x110°).

Les deux diodes lumineuses D1, D2 sont assemblées (par collage conducteur ou soudure) et intégrées dans ledit bloc électronique 3 hermétique. Elles sont donc protégées à l'intérieur du bloc électronique.

De manière non limitative, le montage des diodes dans le bloc électronique 3 peut être réalisé selon plusieurs variantes possibles. Les diodes lumineuses D1, D2 peuvent ainsi être montées sur une ou deux faces d'un substrat du bloc électronique 3 ou sur les deux faces distinctes du substrat du bloc électronique, en fonction du type d'illumination souhaité (par exemple illumination sur un angle de 110° ou de 2x110°).

Sur la figure 9A, le bloc électronique 3a comporte un seul substrat 32 opaque ou transparent et intégrant des "vias" pour transmettre les signaux électriques sur lequel les deux diodes lumineuses D1, D2 sont juxtaposées et interconnectées. Un dépôt conforme 36 de type ALD ("Atomic Layer Deposit") est réalisé sur toute la face de support des diodes pour les encapsuler et les isoler de l'extérieur, permettant de rendre le bloc électronique hermétique.

Sur la figure 9B, le bloc électronique 3b comporte deux substrats 30, 31 (silice ou saphir) opaques ou transparents, comportant des "vias" et des pistes métalliques pour alimenter les diodes lumineuses. Les deux substrats 30, 31 sont assemblés entre eux, grâce à un espaceur 310, par exemple par soudure laser ou brasure métallique et forment un ensemble hermétique dans lequel sont logées les deux diodes lumineuses D1, D2. Les deux diodes lumineuses D1, D2 sont fixées (par exemple par collage conducteur ou brasure) sur la face supérieure du premier substrat 30. Dans la réalisation de la figure 9B, les pistes électriques et les "vias" du circuit sont réalisées sur les deux substrats 30, 31.

Sur la figure 9C, le bloc électronique 3c comporte un seul substrat électronique 32 et un capot 33. Les deux diodes lumineuses sont collées ou soudées sur le substrat électronique 32 et le capot 33, pouvant être transparent, est apposé de manière hermétique sur le substrat pour enfermer lesdites diodes. Dans la réalisation de la figure 9C, les pistes électriques et les "vias" du circuit sont réalisées sur le substrat 32 uniquement.

Les deux diodes lumineuses D1, D2 peuvent être juxtaposées dans un même plan comme sur les figures 9A, 9B ou 9C ou juxtaposées de manière superposée comme sur la figure 9D.

Dans la réalisation de la figure 9D, le bloc électronique 3d comporte également deux substrats électroniques 34, 35 assemblés entre eux pour former un ensemble hermétique dans lequel sont logées les deux diodes lumineuses D1, D2. La première diode D1 peut être fixée sur la face supérieure du premier substrat 34 et la deuxième diode D2 peut être fixée sur la face inférieure du deuxième substrat 35 et positionnée juste au-dessus de la première diode.

Dans les quatre configurations, chaque substrat peut par exemple présenter une longueur de 1000µm alors que chaque diode D1, D2 peut s'étendre sur une longueur inférieure ou égale à 350µm sur un substrat.

Comme indiqué ci-dessus en liaison avec la figure 8A, le bloc électronique 3 du module Mi est connecté à une voie Vi du générateur et à la voie de retour Vn, via les moyens de connexion électrique du module. Le bloc électronique 3 peut comporter des contacts 5 électriques agencés de part et d'autre de chaque bloc 3 et venant pincer chacun un conducteur 14 distinct, un premier conducteur relié au contact électrique d'alimentation dédié Ci_x du module pour se connecter à sa voie Vi d'alimentation et un deuxième conducteur venant se connecter sur la liaison électrique du module qui correspond à la voie de retour Vn menant au générateur IPG.

Dans les quatre configurations, les techniques classiques de microélectronique sont employées pour réaliser les pistes électriques, souder les diodes électroluminescentes et réaliser les connexions électriques nécessaires. Il pourra s'agir de techniques de soudage, brasage, collage, sérigraphie... L'assemblage des diodes sur chaque substrat ainsi que la réalisation de l'ensemble hermétique seront réalisés selon des techniques connues tels que soudure laser, dépôt multicouche de type ALD (pour "Atomic Layer Deposit").

Dans chaque module, le ou les substrats sont orientés suivant une direction parallèle à l'axe du module (et donc à l'axe de la sonde 10 lorsque les modules sont assemblés entre eux). Dans cette configuration, les diodes lumineuses D1, D2 sont orientées pour éclairer suivant des directions transversales par rapport à l'axe de la sonde 10. Au niveau de chaque module Mi, l'illumination peut être réalisée à travers toute la surface latérale de la sonde ou être limitée sur une plage angulaire latérale donnée. Un masquage peut par exemple être réalisée sur la surface latérale de la sonde pour orienter l'illumination dans une ou plusieurs directions latérales données.

Selon les configurations, de manière non limitative, chaque substrat 32 peut être opaque ou au moins partiellement transparent pour laisser passer la lumière émise par les diodes lumineuses sans masquage inapproprié. Le positionnement du masquage dû aux pistes conductrices et l'étendue de transparence de la bague pourront être adaptés à l'orientation des diodes lumineuses et au niveau de diffusion de la lumière. Chaque module peut ainsi être configurée pour créer une illumination directive ou isotrope.

Les figures 10A à 10D illustrent différentes configurations possibles d'illumination. Sur la figure 10A, le module est à deux diodes fixées sur une même face d'un substrat 32 opaque. Le capot 33 est choisi transparent. L'illumination n'est ainsi réalisée que d'un seul côté. Sur la figure 10B, le substrat 32 est transparent et le capot 33 est transparent, permettant d'émettre la lumière des deux côtés. Sur la figure 10C, le module est à deux diodes D1, D2 fixées sur les deux faces opposées d'un même substrat opaque, permettant d'émettre des deux côtés à travers deux capots 33a, 33b transparents. Sur la figure 10D, on peut voir que la configuration de la figure 10C permet d'émettre sur toute la périphérie de la sonde.

Le support des diodes lumineuses D1, D2 est logé dans le boîtier 2 de chaque module Mi et est ensuite enrobé par un matériau d'enrobage type silicone, polyuréthane ou époxy.

Comme illustré par la figure 2, le générateur d'impulsions IPG est configuré pour présenter plusieurs voies Vi, avec i allant de 1 à n. Comme une voie (Vn) du générateur est réservée au signal de retour, le générateur dispose de n-1 voies disponibles pour connecter les modules. Il faut noter qu'il est possible de connecter au moins deux modules en série sur une même voie, comme sur la voie Vn-1 représentée sur la figure 2. A titre d'exemple et de manière non limitative, le générateur peut comporter 8 à 12 voies par canal et jusqu'à 4 canaux indépendants.

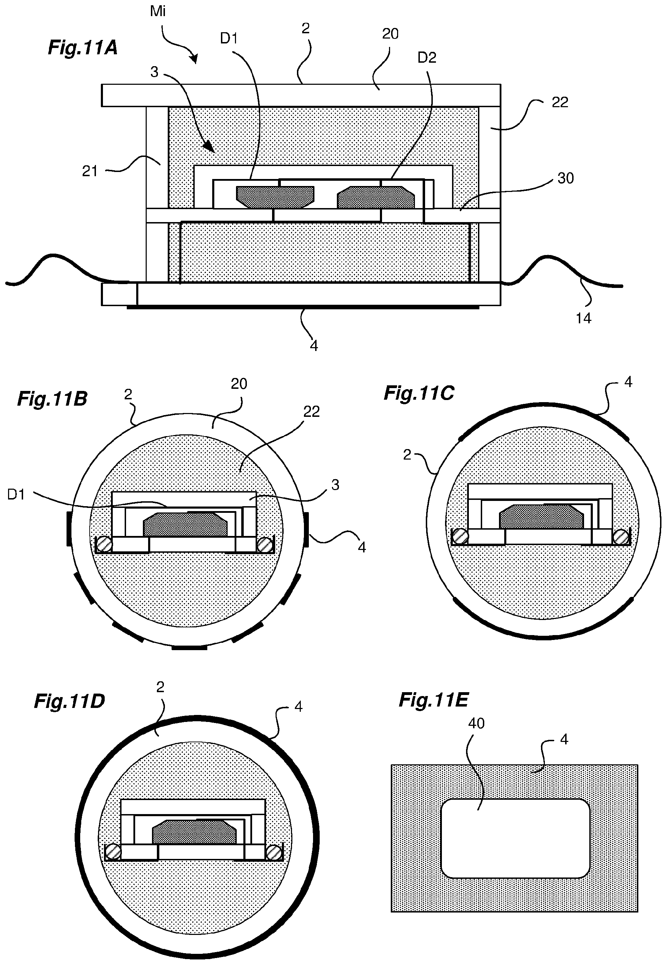

Le module de l'invention peut également être de type hybride, c'est-à-dire portant des moyens de stimulation optique et des moyens de stimulation électrique. Dans ce cas, comme représenté sur les figures 11A et 11B, le module Mi, en plus de porter les diodes lumineuses D1, D2, porte également des électrodes 4 permettant une stimulation électrique des tissus environnants. Ces contacts électriques 4 peuvent être agencés sur au moins une partie de la surface de la paroi latérale du boîtier 2 en cylindre du module Mi. Les électrodes 4 peuvent être connectés au même générateur IPG que celui employé pour alimenter les diodes lumineuses et occuper certaines des voies du générateur. Les électrodes peuvent être réalisée en Platine-Iridium massif ou par dépôt par pulvérisation ou en IrO2 avec dépôt localisé pour laisser passer le faisceau optique. Sur la figure 11B, les électrodes 4 sont des lames plates qui s'étendent suivant une direction parallèle à l'axe de la bague du module. Sur la figure 11C, les électrodes 4 sont en arc de cercle et s'étendent sur une portion angulaire de la bague. Sur la figure 11D, l'électrode 4 est en forme de bague et s'étend sur toute la surface latérale de la bague. Sur la figure 11E, l'électrode est réalisée sous la forme d'un dépôt localisé, permettant de laisser une ouverture 40 pour le faisceau optique.

Lorsqu'un module est hybride, c'est-à-dire qu'il possède à la fois les moyens de stimulation optique et les moyens de stimulation électrique, il faut noter qu'il peut fonctionner selon l'un ou l'autre des deux modes de stimulation ou selon les deux modes de stimulation simultanément.

En référence à la figure 12, dans une même sonde 10, il est possible d'utiliser différents types de modules, c'est-à-dire :
- Module Mopt pouvant fonctionner uniquement en stimulation optique ;
- Module Melec pouvant fonctionner uniquement en stimulation électrique ;
- Module Mh1, Mh2 à stimulation hybride pouvant fonctionner en stimulation électrique et/ou optique ;

Le module à stimulation hybride peut fonctionner de différentes façons. Il peut notamment fonctionner avec une stimulation optique partielle et une stimulation électrique partielle (module Mh2 sur la figure 12) ou avec tous ses moyens de stimulation actifs simultanément (module Mh1 sur la figure 12).

Bien entendu, le module à stimulation hybride peut fonctionner en mode optique et/ou électrique et s'avère plus polyvalent.

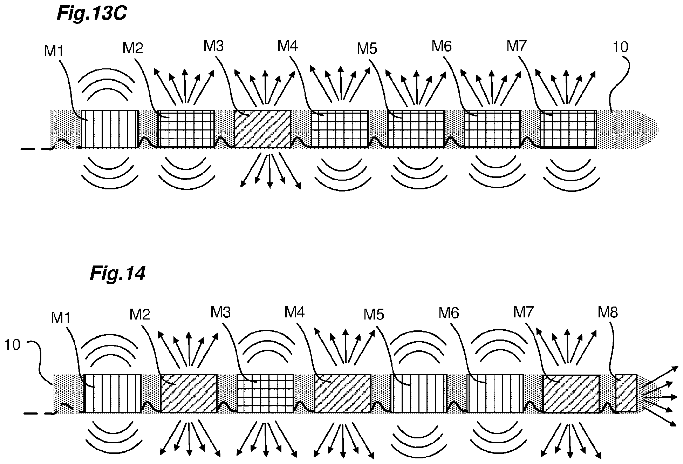

Les figures 13A, 13B et 13C illustrent trois exemples de réalisation d'une sonde de type hybride, pouvant employer ces différents types de modules. Ces exemples sont à considérer de manière non limitative et toute autre combinaison de modules pourrait être proposée.

Sur la figure 13A :
- Des modules (M1, M2 et M3) sont commandés en stimulation électrique uniquement pour réaliser une stimulation de type STN par exemple ;
- Des modules (M4, M5, M6 et M7) sont commandés en stimulation optique uniquement pour réaliser une stimulation de type SNc;

Sur la figure 13B :
- Des modules (M1 et M4) sont commandés en stimulation électrique uniquement ;
- Des modules (M3, M5 et M7) sont commandés en stimulation optique uniquement ;
- Des modules (M2 et M6) sont commandés en stimulation hybride (optique et électrique) ;

Sur la figure 13C :
- Des modules (M2, M4, M5, M6 et M7) sont commandés en stimulation optique sur une première plage angulaire de la sonde et en stimulation électrique sur une deuxième plage angulaire ;

La figure 14 représente un autre mode de réalisation de la sonde 10 de l'invention. Dans cette réalisation, un module M8 à éclairage axial est intégré à l'extrémité distale de la sonde 10. Ce module peut présenter des caractéristiques similaires à celles des modules à illumination latérale décrits ci-dessus. Le support de ses diodes peut être orienté transversalement par rapport à l'axe de la sonde 10.

Il faut noter que l'emploi d'un générateur à impulsions et le montage tête bêche des diodes lumineuses dans chaque module permet de limiter les risques pour le patient en cas de fuites de courant, les courants de fuite devant être limités à 1 µA en moyenne. Ces niveaux de courants de fuite sont difficilement atteignables lorsque l'alimentation électrique est de type continu ou monopolaire.

Par ailleurs, l'utilisation de modules identiques et indépendants à connecter entre eux permet de monter facilement une sonde. Un intégrateur pourra par ailleurs facilement adapter la sonde à ses besoins en sélectionnant le nombre adapté de modules, en sélectionnant ses modules, en fonction de la longueur d'onde à émettre, du nombre de longueurs d'ondes à émettre par module, du type d'illumination à obtenir, du type de stimulation souhaitée (optique et/ou électrique).

## Revendications

1. Module (Mi) à stimulation optique à intégrer dans une sonde implantable dans un être vivant pour illuminer de manière localisée une zone dudit être vivant, ladite sonde étant destinée à comporter une chaîne formée de plusieurs de ces modules, chaque
module comportant :
- un boîtier (2),
- un bloc (3) électronique hermétique logé dans ledit boîtier et comportant deux diodes lumineuses (D1, D2) connectées tête bêche, et
- **caractérisé en ce que** le module comporte :
- des premiers contacts électriques dits amont, agencés sur son boîtier pour se connecter à un premier module à stimulation optique identique adjacent, situé en amont dans une chaîne de modules, et des deuxièmes contacts électriques dits avals agencés sur son boîtier pour se connecter à un deuxième module à stimulation optique identique adjacent, situé en aval dans ladite chaîne de modules,
- des liaisons électriques agencés entre chaque premier contact électrique amont et chaque deuxième contact électrique aval,
- un premier contact électrique d'alimentation dédié (Ci_x), agencé sur son boîtier sur lequel est connecté son bloc électronique.

2. Module selon la revendication 1, lesdites liaisons électriques comportant au moins une liaison formant une ligne de retour électrique commune (Vn) à tous les modules à stimulation optique de la sonde, sur laquelle est connecté ledit bloc électronique du module à stimulation optique.

3. Module selon la revendication 1 ou 2, le bloc électronique (3a, 3b, 3c) comportant au moins un substrat (30, 32) comportant deux faces opposées, lesdites deux diodes lumineuses étant montées sur une seule des deux faces dudit substrat.

4. Module selon la revendication 3, que le bloc électronique (3c) comportant un capot hermétique (33) adapté sur le substrat (32).

5. Module selon la revendication 3, le bloc électronique (3a) comportant un dépôt (36) de type ALD venant recouvrir les deux diodes lumineuses (D1, D2).

6. Module selon la revendication 1, le bloc électronique (3d) comportant deux substrats (34, 35), sur chacun desquels est montée une diode lumineuse distincte.

7. Module selon l'une des revendications 1 à 6, son boîtier (2) comportant une bague (20) fermée à ses deux extrémités par deux bouchons (21), lesdits deux bouchons portant des moyens de support du bloc électronique (3) logé dans le boîtier.

8. Module selon la revendication 7, comportant des électrodes (4) de stimulation sur la surface latérale de sa bague (20).

9. Module selon l'une des revendications 1 à 8, comportant un
matériau d'enrobage (22) injecté dans son boîtier (2) autour du bloc électronique (3) hermétique.

10. Sonde implantable dans un être vivant destinée à être connectée électriquement à une source d'alimentation électrique et présentant une architecture allongée, comportant plusieurs modules (Mi) à stimulation optique juxtaposés le long de la sonde et séparés entre eux d'une distance non nulle, ladite sonde comprenant un matériau d'enrobage (12) venant combler l'espace entre deux modules adjacents, chaque module étant tel que défini dans l'une des revendications 1 à 9, ladite sonde comportant plusieurs voies électriques destinées chacune à être raccordé électriquement en point à point à une voie électrique distincte de la source d'alimentation électrique, chaque module (Mi) à stimulation optique de la sonde étant connectée en série sur une voie électrique distincte de la sonde via son contact électrique d'alimentation dédié (Ci_x).

11. Sonde selon la revendication 10, comportant une
armature (11) formée d'un câble sur lequel sont enfilées lesdits modules (Mi).

12. Sonde selon la revendication 11, comportant, entre deux modules à
stimulation optique adjacents, la sonde comporte une bague de séparation (13) mécanique, enfilée sur ladite armature (11).

13. Sonde selon l'une des revendications 10 à 12, comportant un module à illumination axiale situé à proximité de l'extrémité distale de la sonde (10).

14. Dispositif (1) d'illumination implantable, destiné à être implanté dans un être vivant pour illuminer de manière localisée une zone dudit être vivant, ledit dispositif comportant une source d'alimentation électrique comportant plusieurs voies (Vi) d'alimentation électrique en parallèle et une sonde (10) connectée électriquement à la source d'alimentation électrique et présentant une architecture allongée entre une extrémité proximale et une extrémité distale, ledit dispositif étant où ladite sonde (10) est telle que définie dans l'une des revendications 10 à 13.

15. Dispositif selon la revendication 14, où la source d'alimentation
électrique est un générateur d'impulsions implantable (IPG).

## Patentansprüche

1. Modul (Mi) mit optischer Stimulation zum Einbau in eine in ein Lebewesen implantierbare Sonde, um einen Bereich des Lebewesens örtlich begrenzt zu beleuchten, wobei die Sonde dazu bestimmt ist, eine von mehreren dieser Module gebildete Kette aufzuweisen, wobei jedes Modul aufweist:
- ein Gehäuse (2),
- einen hermetisch dichten Elektronikblock (3), der im Gehäuse untergebracht ist und zwei Kopf bei Fuß verbundene Leuchtdioden (D1, D2) aufweist, und
**dadurch gekennzeichnet, dass** das Modul aufweist:
- erste so genannte stromaufwärts liegende elektrische Kontakte, die auf seinem Gehäuse angeordnet sind, um sich mit einem benachbarten gleichen ersten Modul mit optischer Stimulation zu verbinden, das sich stromaufwärts in einer Kette von Modulen befindet, und zweite so genannte stromabwärts liegende elektrische Kontakte, die auf seinem Gehäuse angeordnet sind, um sich mit einem benachbarten gleichen zweiten Modul mit optischer Stimulation zu verbinden, das sich stromabwärts in der Kette von Modulen befindet,
- elektrische Verbindungen, die zwischen jedem ersten stromaufwärts liegenden elektrischen Kontakt und jedem zweiten stromabwärts liegenden elektrischen Kontakt eingerichtet sind,
- einen auf seinem Gehäuse eingerichteten dedizierten ersten elektrischen Versorgungskontakt (Ci_x), auf dem sein Elektronikblock verbunden ist.

2. Modul nach Anspruch 1, wobei die elektrischen Verbindungen mindestens eine Verbindung aufweisen, die eine allen Modulen mit optischer Stimulation der Sonde gemeinsame elektrische Rückleitung (Vn) bildet, auf der der Elektronikblock des Moduls mit optischer Stimulation verbunden ist.

3. Modul nach Anspruch 1 oder 2, wobei der Elektronikblock (3a, 3b, 3c) mindestens ein zwei gegenüberliegende Seiten aufweisendes Substrat (30, 32) aufweist, wobei die zwei Leuchtdioden auf eine einzige der zwei Seiten des Substrats montiert sind.

4. Modul nach Anspruch 3, wobei der Elektronikblock (3c) eine an das Substrat (32) angepasste hermetisch dichte Haube (33) aufweist.

5. Modul nach Anspruch 3, wobei der Elektronikblock (3a) eine Beschichtung (36) des Typs ALD aufweist, die die zwei Leuchtdioden (D1, D2) bedeckt.

6. Modul nach Anspruch 1, wobei der Elektronikblock (3d) zwei Substrate (34, 35) aufweist, auf jedes von denen eine unterschiedliche Leuchtdiode montiert ist.

7. Modul nach einem der Ansprüche 1 bis 6, wobei sein Gehäuse (2) einen an seinen zwei Enden durch zwei Verschlusskappen (21) verschlossenen Ring (20) aufweist, wobei die zwei Verschlusskappen Stützeinrichtungen des im Gehäuse untergebrachten Elektronikblocks (3) tragen.

8. Modul nach Anspruch 7, das Stimulationselektroden (4) auf der Seitenfläche seines Rings (20) aufweist.

9. Modul nach einem der Ansprüche 1 bis 8, das ein Umhüllungsmaterial (22) aufweist, das in sein Gehäuse (2) um den hermetisch dichten Elektronikblock (3) herum eingespritzt wird.

10. In ein Lebewesen implantierbare Sonde, die dazu bestimmt ist, elektrisch mit einem Stromversorgungsgerät verbunden zu werden, und eine längliche Architektur aufweist, die mehrere Module (Mi) mit optischer Stimulation aufweist, die entlang der Sonde nebeneinander angeordnet und durch einen Abstand ungleich Null voneinander getrennt sind, wobei die Sonde ein Umhüllungsmaterial (12) enthält, das den Raum zwischen zwei benachbarten Modulen ausfüllt, wobei jedes Modul wie in einem der Ansprüche 1 bis 9 definiert ist, wobei die Sonde mehrere Stromkanäle aufweist, die je dazu bestimmt sind, Punkt-zu-Punkt an einen unterschiedlichen Stromkanal des Stromversorgungsgeräts elektrisch angeschlossen zu werden, wobei jedes Modul (Mi) mit optischer Stimulation der Sonde in Reihe auf einem unterschiedlichen Stromkanal der Sonde über seinen dedizierten elektrischen Versorgungskontakt (Ci_x) verbunden ist.

11. Sonde nach Anspruch 10, die eine Armatur (11) aufweist, die von einem Kabel gebildet wird, auf das die Module (Mi) aufgeschoben sind.

12. Sonde nach Anspruch 11, die zwischen zwei benachbarten Modulen mit optischer Stimulation einen mechanischen Trennring (13) aufweist, der auf die Armatur (11) aufgeschoben ist.

13. Sonde nach einem der Ansprüche 10 bis 12, die ein Modul mit axialer Beleuchtung aufweist, das sich in der Nähe des distalen Endes der Sonde (10) befindet.

14. Implantierbare Beleuchtungsvorrichtung (1), die dazu bestimmt ist, in ein Lebewesen implantiert zu werden, um einen Bereich des Lebewesens örtlich begrenzt zu beleuchten, wobei die Vorrichtung ein Stromversorgungsgerät aufweist, das mehrere parallele Stromversorgungskanäle (Vi) und eine Sonde (10) aufweist, die elektrisch mit dem Stromversorgungsgerät verbunden ist und zwischen einem proximalen Ende und einem distalen Ende eine längliche Architektur aufweist, wobei die Sonde (10) wie in einem der Ansprüche 10 bis 13 definiert ist.

15. Vorrichtung nach Anspruch 14, wobei das Stromversorgungsgerät ein implantierbarer Impulsgenerator (IPG) ist.

## Claims

1. Optically stimulating module (Mi) to be integrated into a probe that is implantable into a living being with a view to locally illuminating a region of said living being, said probe being intended to comprise a chain formed from a plurality of these modules, each module comprising:
- a casing (2),
- a hermetic electronic unit (3) housed in said casing and comprising two luminous diodes (D1, D2) connected back-to-back, and
- **characterized in that** the module comprises:
- first electrical contacts, which are referred to as upstream electrical contacts, arranged on their casing so as to connect to a first identical adjacent optically stimulating module located upstream in a chain of modules, and second electrical contacts, which are referred to as downstream electrical contacts, arranged on their casing so as to connect to a second identical adjacent optically stimulating module located downstream in said chain of modules,
- electrical links arranged between each upstream first electrical contact and each downstream second electrical contact,
- a first dedicated electrical supply contact (Ci_x), arranged on its casing, to which its electronic unit is connected.

2. Module according to Claim 1, said electrical links comprising at least one link forming an electrical return line (Vn) common to all the optically stimulating modules of the probe, to which link said electronic unit of the optically stimulating module is connected.

3. Module according to Claim 1 or 2, the electronic unit (3a, 3b, 3c) comprising at least one substrate (30, 32) comprising two opposite faces, said two luminous diodes being mounted on a single one of the two faces of said substrate.

4. Module according to Claim 3, the electronic unit (3c) comprising a suitable hermetic cover (33) on the substrate (32).

5. Module according to Claim 3, the electronic unit (3a) comprising a deposit (36) produced by ALD covering the two luminous diodes (D1, D2).

6. Module according to Claim 1, the electronic unit (3d) comprising two substrates (34, 35), on each of which one separate luminous diode is mounted.

7. Module according to one of Claims 1 to 6, its casing (2) comprising a ring (20) that is closed at its two ends by two plugs (21), said two plugs bearing means for holding the electronic unit (3) housed in the casing.

8. Module according to Claim 7, comprising stimulating electrodes (4) on the lateral surface of its ring (20).

9. Module according to one of Claims 1 to 8, comprising a coating material (22) injected into its casing (2) around the hermetic electronic unit (3).

10. Probe that is implantable into a living being, said probe being intended to be electrically connected to an electrical power source and having an elongate architecture, said probe comprising a plurality of optically stimulating modules (Mi) juxtaposed along the probe and separated from each other by a non-zero distance, said probe comprising a coating material (12) that fills the space between two adjacent modules, each module being such as defined in one of Claims 1 to 9, said probe comprising a plurality of electrical paths each intended to be electrically connected, point-to-point, to one separate electrical path of the electrical power source, each optically stimulating module (Mi) of the probe being connected in series on a separate electrical path of the probe via its dedicated electrical supply contact (Ci_x).

11. Probe according to Claim 10, comprising a reinforcement (11) formed of a wire over which said modules (Mi) are slipped.

12. Probe according to Claim 11, comprising, between two adjacent optically stimulating modules, a mechanically dividing ring (13) slipped over said reinforcement (11).

13. Probe according to one of Claims 10 to 12, comprising an axially illuminating module located in proximity to the distal end of the probe (10).

14. Implantable illuminating device (1) intended to be implanted into a living being with a view to locally illuminating a region of said living being, said device comprising an electrical power source comprising a plurality of parallel electrical supply paths (Vi) and a probe (10) that is electrically connected to the electrical power source and that has an elongate architecture between a proximal end and a distal end, wherein said probe (10) is such as defined in one of Claims 10 to 13.

15. Device according to Claim 14, wherein the electrical power source is an implantable pulse generator (IPG).
